# EUROPEAN PATENT APPLICATION

(11) **EP 1 714 967 A1**
(43) Date of publication of application: **25.10.2006**
(21) Application number: 05008862.4
(22) Date of filing: 22.04.2005
(51) Int. Cl.: C07D 493/18, A61K 31/335, A61P 33/06

(54) **Antiparasitic artemisinin derivatives**

(71) Applicant: Bayer HealthCare AG, 51368 Leverkusen (DE)
(72) Inventor: Schmeer, Karl, Dr., 40764 Langenfeld (DE); Wollborn, Ute, Dr., 47800 Krefeld (DE)

(57) **Abstract**

The invention relates to derivatives of artemisinin according to formula (I) and processes for their preparation and their use for preparing medicaments for the treatment and/or prophylaxis of disorders, especially of malaria. 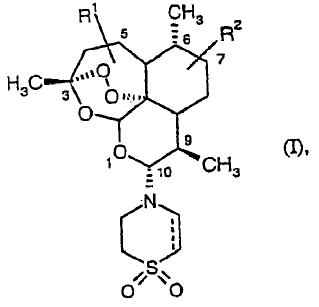 in which
...... represents a single or a double bond,
R¹ represents hydrogen and hydroxy,
and
R² represents hydrogen or hydroxy,
wherein
...... represents a double bond if R¹ and R² are hydrogen.
Or one of its salts, its solvates or the solvates of its salts.

## Description

The invention relates to novel derivatives of artemisinin and processes for their preparation and their use for preparing medicaments for the treatment and/or prophylaxis of disorders, especially of malaria.

Malaria is the most important human parasitic disease in the world today. Approximately 270 million people throughout the world are infected with malaria, with about 2 million dying each year. The ability of parasites to produce a complex survival mechanism by expressing variant antigens on the surface of infected erythrocytes makes it possible for the parasites to escape from the destructive action of the host immune response against these antigens. In addition, the increasing rate of malaria infection is due to the spread of chloroquine-resistant strains of Plasmodium falciparum and the other multi-drug resistant strains.

In the field of animal health, parasitic diseases are a major problem, especially those diseases which are functionally related to malaria. For instance, neosporosis is a term used to describe diseases caused by parasites of the species Neospora, especially Neospora caninum, in animals. Neospora infections are known to occur in dogs, cattle, sheep, goats and horses.

The final host for Neospora spp., including Neospora caninum, is unknown and, in addition, the complete cycle of development of the parasite is not understood. The asexual phases of reproduction, known as schizogony, and the behaviour of the unicellular tachyzoite/bradyzoite stage have been clarified, however. Tachyzoites are infectious unicellular parasite stages of about 3-7 x 1-5 mm in size formed after intracellular reproduction termed endodyogeny. Reproduction via tachyzoites takes place preferentially in organelles such as muscle or nerve cells. Pathological symptoms invoked after an infection are associated mainly in those tissues. Some five to six weeks after natural infection in a dog, symptoms of the disease are hypersensitivity caused by inflammation of neuronal cells and increasing tendency to hyperextension of the hind legs. Histopathological lesions are apparent in the nervous system, preferentially in the brain and spinal cord. Extensive non-suppurative inflammations, glial excrescences and perivascular infiltrations of mononuclear cells (macrophages, lymphocytes, plasma cells) dominate, and are also partly apparent in eosinophils and neutrophils. In the muscular system, macroscopically observable necroses and degenerative changes appear. Apart from the more or less strongly developed atrophy, long pale longitudinal stripes are evident.

In California and Australia, Neospora caninum infections appear to be the main cause for abortion in cattle. Symptoms of the disease in cattle are similar to those in the dog. Ataxia is apparent, joint reflexes are weakened and pareses at the hind legs, partly in all four legs, can be observed. The histological picture is similar to that of the dog; mainly non-suppurative meningitis and myelitis.

Data on in vivo activity of compounds suitable against neosporosis are rare because adequate in vivo test systems still have to be developed. Sulfadiazin (administered via drinking water) is effective in experimentally infected mice, only if the treatment was prophylactic, that is, the treatment was started before infection. In dogs, treatment with sulfadiazin and clindamycin is only successful if it is started early, that is, at the appearance of first clinical symptoms as a result of neuronal inflammation.

Coccidiosis, an infection of the small intestine, is relatively rarely diagnosed in humans, where it is caused by Isospora belli. However, humans are also the final host of at least two cyst-forming coccidial species (Sarcocystis suihominis and S. bovihominis). Consumption of raw or inadequately cooked pork or beef containing such cysts can lead to severe diarrhoea, the cause of which is probably seldom diagnosed correctly. Coccidia (phylum Apicomplexa, suborder Eimeriina) are one of the most successful groups of parasitic protozoans, having conquered virtually every class of Metazoa. The ones that are of particular importance for man are the 60-100 species which parasitise domestic animals and which in some instances can cause very severe losses, especially in poultry, although also in lambs, calves, piglets, rabbits and other animals (see Table A).

**Table A: Causatives of intestinal coccidiosis in domestic animals**

| Animal | number of Eimeria and/or Isospora species*) | most pathogenic and/or very common species (E=Eimeria, I=Isospora) |
|---|---|---|
| chicken (Gallus gallus) | 7 | E.tenella, E.necatrix, E.maxima, E.acervulina |
| turkey (Meleargidis gallopavo) | 7 | E.meleagrimitis, E.adenoides |
| goose (Anser anser) | 6 | E.anseris, E.truncata, E.nocens, E. kotlani |
| duck (Anas platyhynehus) | 3 | Tyzzeria pemiciosa, E.anatis |
| pigeon (Columba livia) | 2 | E.columbarum, E.labbeanea |
| rabbit (Oryctolagus cuniculus) | 11(12) | E.intestinalis, E.flavescens, E.stiedai, E.magna, E.perforans |
| sheep (Ovis arius) | 11(16) | E.ovinoidalis, E.ashata, E.ovina |
| goat (Capra hircus) | 12(15) | E.ninakohlyakimovae,E.arloingi |
| cattle (Bos taurus) | 12(15) | E.zuemii, E.bovis, E.auburnensis |
| pig (Sus scofra) | 7(14) | I.suis, E.debliecki, E.scabra |
| dog (Canis familiaris) | 5 | I.canis, I.(Cystisospora)burrowsi |
| cat (Felis catus) | 2+6 | I.felis, I.rivolta as final host: Sarcocystis bovifelis, S.ovifelis, S.fusiformis, S.muris, S.cuniculi, Toxoplasma gondii |

| | | |
|---|---|---|
| *) regarding to Pellerdy (1974), Eckert et al, (1995b, Levine and Ivens (1970) and Mehlhorn 1988) | | |

Most of the pathogenic species are strictly host-specific. They have a complex life cycle with two asexual reproduction phases (schizogony or merogony, and sporogony) and a sexual development phase (gametogony). In view of the major importance of coccidiosis, numerous reviews are available, for instance, by Davies et al. (1963), Hammond and Long (1973), Long (1982, 1990), and Pellerdy (1974). The economically important species sometimes differ very considerably in their sensitivity to medicinal active ingredients. The sensitivity of the different developmental stages to medicinal agents also varies enormously.

As far as the use of drugs is concerned, prophylaxis is the main approach in poultry, in which symptoms do not appear until the phase of increased morbidity, and therapy is the principal strategy in mammals (McDougald 1982). Polyether antibiotics and sulfonamides, among other drugs, are currently used for such treatment and prophylaxis. However, drug-resistant strains of Eimeria have emerged and drug-resistance is now a serious problem. New drugs are therefore urgently required. Given the multiplicity of pathogens and hosts, there is no "ideal model" for identifying and testing anticoccidial agents. For example, most of the many substances used for preventing coccidiosis in poultry are insufficiently effective or even completely ineffective against mammalian coccidia (Haberkorn and Mundt; 1989; Haberkorn 1996). Numerous works and sets of instructions have been published on testing of active ingredients in animals for anticoccidial efficacy, for immunisation, etc. One particularly important and comprehensive example is the survey of current methods published by Eckert et al. (1995a).

The compound artemisinin, also known as qinghaosu (A), is a tetracyclic 1,2,4-trioxane occurring in Artemisia annua. Artemisinin and its derivatives dihydroartemisinin (B), artemether (C) and sodium artesunate (D) have been used for the treatment of malaria.

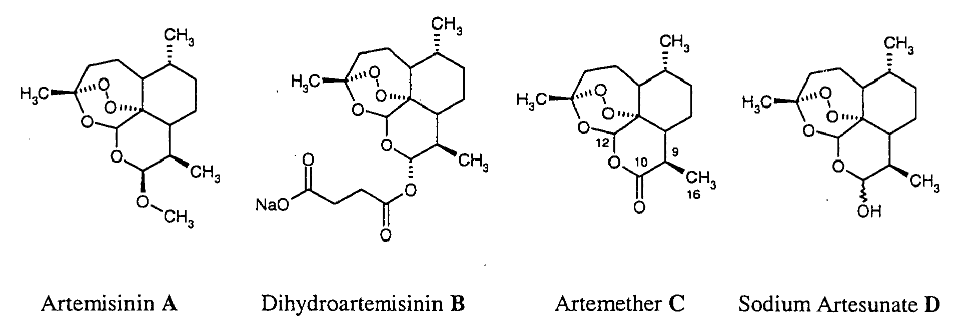

Different modes of action have been proposed by various groups to account for the action of artemisinin and its derivatives in treating malaria (Posner et al., J. Am. Chem. Soc. 1996, 118, 3537*;* Posner et al., J. Am. Chem. Soc. 1995, 117, 5885; Posner et al., J. Med. Chem. 1995, 38, 2273*)*. However, irrespective of actual mode of action, all current derivatives suffer from poor oral bioavailability and poor stability (Meshnick et al., Parasitology Today 1996, 12, 79*),* especially the 'first generation' ethers and esters artemether and sodium artesunate obtained from dihydroartemisinin. Extensive chemical studies carried out on artemisinin and derivatives indicate that a cause of instability is the facile opening of the trioxane moiety in artemisinin itself, or in the metabolite common to all currently used derivatives artemether, arteether and artesunate, namely dihydroartemisinin. Ring opening will provide the free hydroperoxide, which is susceptible to reduction. Removal of this group ensures destruction of drug activity with the reduction products being transformed into desoxo metabolites. In order to render ring-opening less facile, the oxygen atom at C-10 can be either removed to provide 10-deoxydihydroartemisinin, or replaced by other groups, and this has provided the basis for the so-called 'second generation' compounds which are generally 10-deoxy artemisinin derivatives. In addition, derivatives of artemisinin have also been prepared with a variety of substituents at C-9.

Artemisinin derivatives are also known in which the oxygen atom at C-10 has been replaced by an amine group. For instance, Yang et al (Biorg. Med. Chem. Lett., **1995,** 5, 1791-1794) synthesised ten new artemisinin derivatives in which the oxygen atom at C-10 was replaced by a group -NHAr where Ar represents a phenyl, 3-chlorophenyl, 4-chlorophenyl, 3-bromophenyl, 4-bromophenyl, 4-iodophenyl, 4-methylphenyl, 4-methoxyphenyl, 3-carboxylphenyl or 4-carboxylphenyl group. These compounds were tested for in vivo activity against the K173 strain of Plasmodium berghei and found to be active.

WO 00/04024, WO 03/035651, WO 03/048167, WO 03/048168 and WO 03/076446 disclose further C-10 substituted derivatives of artemisinin.

Whilst the current artemisinin derivatives are successful, there are problems associated with stability, bioavailability and potential neurotoxicity. There is also a need for artemisinin derivatives which exhibit a broad spectrum of activity against a variety of parasites.

It has now been discovered that certain C-10 substituted derivatives of artemisinin which are hydroxy substituted and/or have a carbon-carbon double bond in the C-10 substituent are effective in the treatment of diseases caused by infection with a parasite.

The present invention relates to compounds of the formula 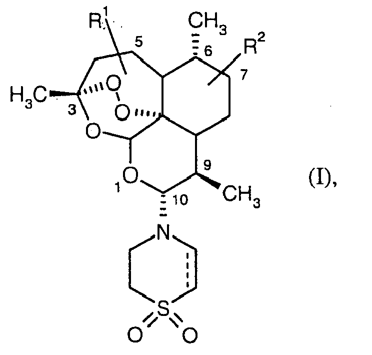 in which
- .....: represents a single or a double bond,
- R¹: represents hydrogen or hydroxy,
and
- R²: represents hydrogen or hydroxy,
wherein
..... represents a double bond if R¹ and R² are hydrogen.

The compounds according to the invention can also be present in the form of their salts, solvates or solvates of the salts.

Depending on their structure, the compounds according to the invention can exist in stereoisomeric forms (enantiomers, diastereomers). The invention therefore relates to the enantiomers or diastereomers and to their respective mixtures. Such mixtures of enantiomers and/or diastereomers can be separated into stereoisomerically unitary constituents in a known manner.

The invention also relates to tautomers of the compounds, depending on the structure of the compounds.

Salts for the purposes of the invention are preferably physiologically acceptable salts of the compounds according to the invention.

Physiologically acceptable salts of the compounds (I) include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, propionic acid, lactic acid, tartaric acid, malic acid, citric acid, fumaric acid, maleic acid and benzoic acid.

Physiologically acceptable salts of the compounds (I) also include salts of customary bases, such as for example and preferably alkali metal salts (for example sodium and potassium salts, alkaline earth metal salts (for example calcium and magnesium salts) and ammonium salts derived from ammonia or organic amines having 1 to 16 carbon atoms, such as illustratively and preferably ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, dihydroabietylamine, arginine, lysine, ethylenediamine and methylpiperidine.

Solvates for the purposes of the invention are those forms of the compounds that coordinate with solvent molecules to form a complex in the solid or liquid state. Hydrates are a specific form of solvates, where the coordination is with water.

Preference is given to compounds of the formula (I)
in which
- .....: represents a single or a double bond,
- R¹: represents hydroxy,
and
- R²: represents hydrogen,
or
- .....: represents a single or a double bond,
- R¹: represents hydrogen,
and
- R²: represents hydroxy,
or
- .....: represents a double bond,
- R¹: represents hydrogen,
and
- R²: represents hydrogen.
Preference is also given to compounds of the formula (I) in which R¹ represents hydroxy and the hydroxy-group is linked to the 5 position.

Preference is also given to compounds of the formula (I) in which R² represents hydroxy and the hydroxy-group is linked to the 7 position.

Very particular preference is given to the compounds
4-[(3R,6R,9R,10R,12R,12aR)-3,6,9-trimethyldecahydro-3,12-epoxy[1,2]dioxepino[4,3-i]-isochromen-10-yl]-3,4-dihydro-2H-1,4-thiazine-1,1-dioxide 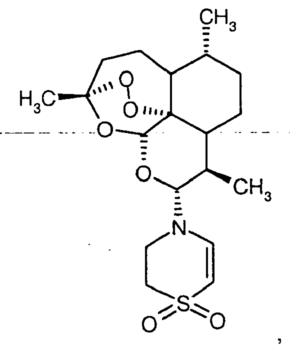 (3R,5S,6R,9R,10R,12R,12aS)-10-(1,1-dioxidothiomorpholin-4-yl)-3,6,9-trimethyl-decahydro-3,12-epoxy[1,2]dioxepino[4,3-i]isochromen-5-ol 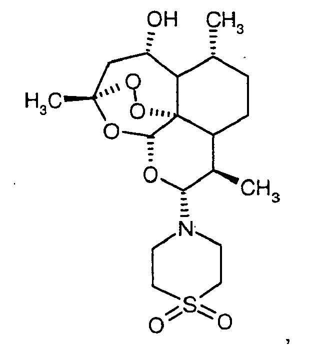 (3R,6S,7R,9R,10R,12R,12aR)-10-(1,1-dioxidothiomorpholin-4-yl)-3,6,9-trimethyl-decahydro-3,12-epoxy[1,2]dioxepino[4,3-i]isochromen-7-ol 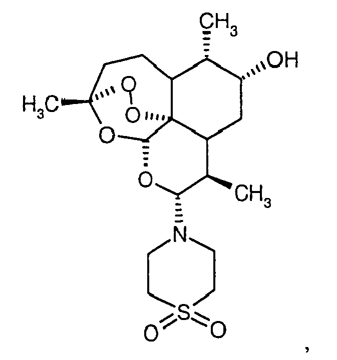 (3R,5S,6R,9R,10R,12R,12aS)-10-(1,1-dioxido-2,3-dihydro-4H-1,4-thiazin-4-yl)-3,6,9-trimethyldecahydro-3,12-epoxy[1,2]dioxepino[4,3-i]isochromen-5-ol 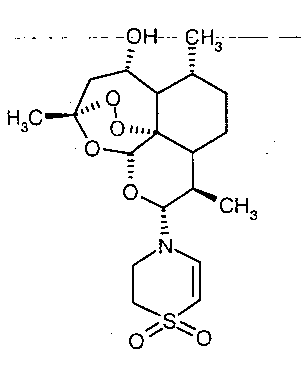 and
(3R,6S,7R,9R,10R,12R,12aR)-10-(1,1-dioxido-2,3-dihydro-4H-1,4-thiazin-4-yl)-3,6,9-trimethyldecahydro-3,12-epoxy[1,2]dioxepino[4,3-i]isochromen-7-ol

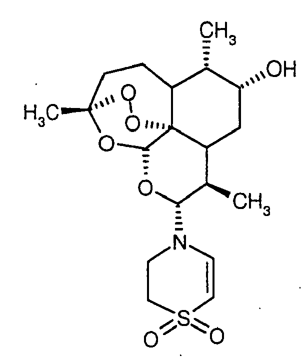

Very particular preference is also given to combinations of two or more of the above mentioned preferences.

The invention further relates to a process for preparing the compounds of the formula (I), characterized in that a compound of the formula 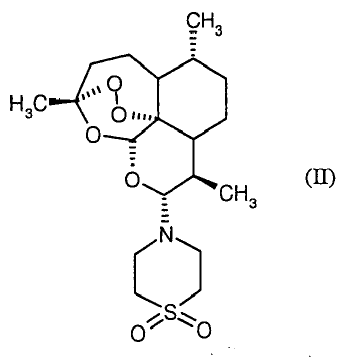 is incubated with liver microsomes of different species including man.

The compound of the formula (II) can be prepared as describes in WO 00/04024.

The preparation of the compounds according to the invention can be illustrated by means of the following synthetic scheme:

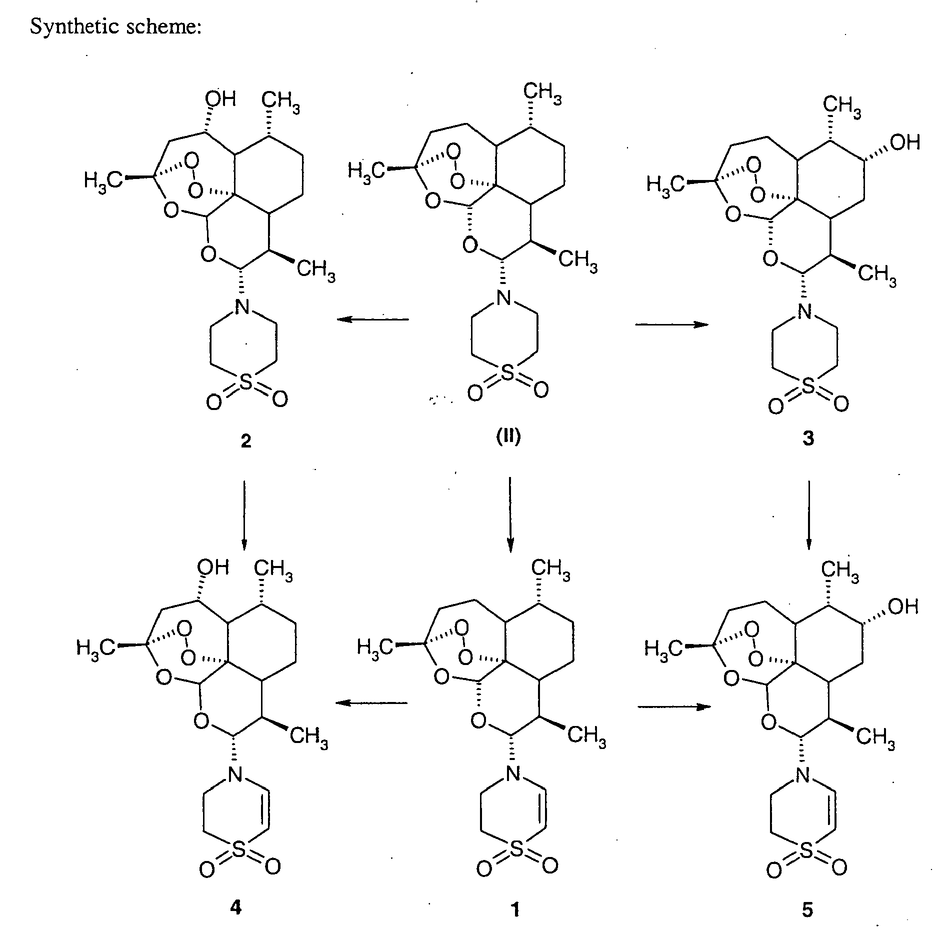

The compounds according to the invention exhibit an unforeseeable, useful pharmacological and pharmacokinetic activity spectrum. They are effective in the treatment of diseases caused by infection with a parasite, in particular a parasite of the genera Plasmodium, Neospora or Eimeria, especially Plasmodium falciparum, Neospora caninum and Eimeria tenella which cause malaria, neosporosis and coccidiosis respectively.

They are therefore suitable for use as medicaments for the treatment and/or prophylaxis of disorders in humans and animals.

The compounds according to the invention are because of their pharmacological properties useful alone or in combination with other active components for treating and/or preventing malaria.

The present invention also includes a pharmaceutical composition as described hereinafter for use in the treatment and/or prophylaxis of a disease caused by infection with a parasite. Preferably, the parasite is an organism of the genus Plasmodium, the genus Neospora, or the genus Eimeria.

The present invention also provides a method for treating a disease caused by infection with a parasite which comprises administering to a host in need of such treatment a therapeutically effective amount of a compound of the general formula (I) as defined above. Preferably, the parasite is an organism of the genus Plasmodium, the genus Neospora, or the genus Eimeria.

The present invention further provides medicaments containing at least one compound according to the invention, preferably together with one or more pharmacologically safe excipients, and also their use for the above mentioned purposes.

The active component can act systemically and/or locally. For this purpose, it can be applied in a suitable manner, for example orally, parenterally, pulmonally, nasally, sublingually, lingually, buccally, rectally, transdermally, conjunctivally, otically or as an implant.

For these application routes, the active component can be administered in suitable application forms.

Useful oral application forms include application forms which release the active component rapidly and/or in modified form, such as for example tablets (non-coated and coated tablets, for example with an enteric coating), capsules, sugar-coated tablets, granules, pellets, powders, emulsions, suspensions, solutions and aerosols.

Parenteral application can be carried out with avoidance of an absorption step (intravenously, intraarterially, intracardially, intraspinally or intralumbarly) of with inclusion of an absorption (intramuscularly, subcutaneously, intracutaneously, percutaneously or intraperitoneally). Useful parenteral application forms include injection and infusion preparations in the form of solutions, suspensions, emulsions, lyophilisates and sterile powders.

Forms suitable for other application routes include for example inhalatory pharmaceutical forms (including powder inhalers, nebulizers), nasal drops/solutions, sprays; tablets or capsules to be administered lingually, sublingually or buccally, suppositories, ear and eye preparations, vaginal capsules, aqueous suspensions (lotions, shake mixtures), lipophilic suspensions, ointments, creams, milk, pastes, dusting powders or implants.

The active components can be converted into the recited application forms in a manner known per se. This is carried out using inert non-toxic, pharmaceutically suitable excipients. Pharmacologically safe excipients include inter alia carriers (for example microcrystalline cellulose), solvents (for example liquid polyethylene glycols), emulsifiers (for example sodium dodecyl sulphate), dispersing agents (for example polyvinylpyrrolidone), synthetic and natural biopolymers (for example albumin), stabilizers (for example antioxidants such as ascorbic acid), colorants (for example inorganic pigments such as iron oxides) or taste and/or odor corrigents.

For treatment of and prophylaxis against coccidiosis and related parasites, for instance, in poultry, especially in chickens, ducks, geese and turkeys, 0.1 to 100 ppm, preferably 0.5 to 100 ppm of the active compound may be mixed into an appropriate, edible material, such as nutritious food. If desired, the amounts applied can be increased, especially if the active compound is well tolerated by the recipient. Accordingly, the active compound can be applied with the drinking water.

For the treatment of a single animal or a human, for instance, for the treatment of coccidiosis in mammals or toxoplasmosis, amounts of 0.5 to 100 mg/kg body weight active compound are preferably administered daily to obtain the desired results. Nevertheless, it may be necessary from time to time to depart from the amounts mentioned above, depending on the body weight of the mammal, the method of application and its individual reaction to the drug or the kind of formulation or the time or interval in which the drug is applied. In special cases, it may be sufficient to use less than the minimum amount given above, whilst in other cases the maximum dose may have to be exceeded. For a larger dose, it may be advisable to divide the dose into several smaller single doses.

The percentages in the tests and examples which follows are, unless otherwise stated, by weight; parts are by weight. Solvent ratios, dilution ratios and concentrations reported for liquid/liquid solutions are each based on the volume.

### A. Examples

### Operative examples

### Example 1

4-[(3R,6R,9R,10R,12R,12aR)-3,6,9-trimethyldecahydro-3,12-epoxy[1,2]dioxepino[4,3-i]-isochromen-10-yl]-3,4-dihydro-2H-1,4-thiazine-1,1-dioxide

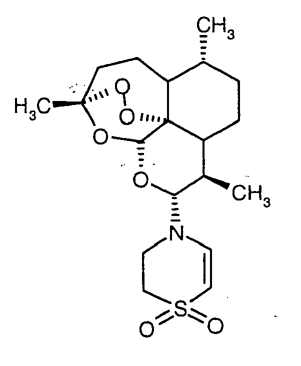

### Example 2

(3R,5S,6R,9R,10R,12R,12aS)-10-(1,1-dioxidothiomorpholin-4-yl)-3,6,9-trimethyl-decahydro-3,12-epoxy[1,2]dioxepino[4,3-i]isochromen-5-ol

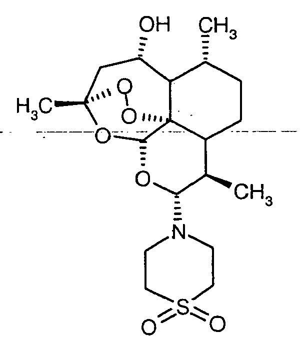

### Example 3

(3R,6S,7R,9R,10R,12R,12aR)-10-(1,1-dioxidothiomorpholin-4-yl)-3,6,9-trimethyl-decahydro-3,12-epoxy[1,2]dioxepino[4,3-i]isochromen-7-ol

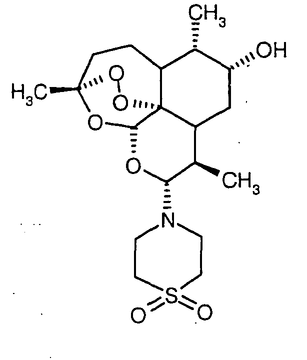

### Example 4

(3R,5S,6R,9R,10R,12R,12aS)-10-(1,1-dioxido-2,3-dihydro-4H-1,4-thiazin-4-yl)-3,6,9-trimethyldecahydro-3,12-epoxy[1,2]dioxepino[4,3-i]isochromen-5-ol

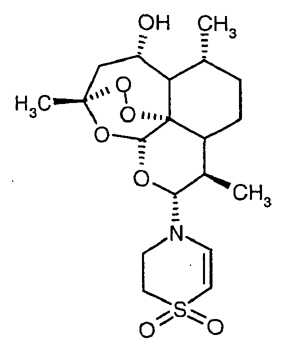

### Example 5

(3R,6S,7R,9R,10R,12R,12aR)-10-(1,1-dioxido-2,3-dihydro-4H-1,4-thiazin-4-yl)-3,6,9-trimethyldecahydro-3,12-epoxy[1,2]dioxepino[4,3-i]isochromen-7-ol

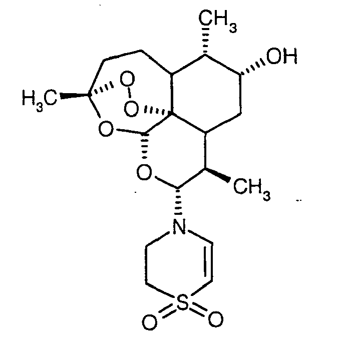

**Table 1: ¹H-Chemical shifts (ppm) of examples 1 to 5, as measured at 700.4 MHz, at 298 K in CDCl₃ (The protons are named by the C-atoms.)**

| **C-Atoms** | **Example 1** | **Example 2** | **Example 3** | **Example 4** | **Example 5** |
|---|---|---|---|---|---|
| C2 | 3.18/5.22 | 3.15/3.28 | 3.15/3.28 | 3.0/3.28/3.40/ 3.72/3.85 | 3.32/3.90/ 4.08/ |
| C3 | 3.88/4.09/6.55 | 3.38/3.52 | 3.42/3.52 | | |
| C3' | - | - | - | - | - |
| C4' | 2.07/2.40 | 2.55 / 2.55 | 2.06/2.41 | 2.51 | 2.06/2.45 |
| C5' | 1.52 / 1.94 | 3.98 | 1.50/1.84 | 3.98 | 1.52 / 1.84 |
| C5'a | 1.28 | ~1.34 | 1.84 | 1.38 | 1.84 |
| C6' | 1.37 | 1.55 | 1.55 | 1.55 | 1.55 |
| C7' | 1.07 / 1.78 | 1.12/1.76 | 3.86 | 1.15 / 1.80 | 3.88 |
| C8' | 1.35 / 1.78 | 1.38 / 1.76 | 1.56/1.94 | 1.37 / 1.80 | ~1.60 / 1.95 |
| C8'a | 1.67 | 1.67 | 2.17 | 1.69 | 2.25 |
| C9' | 2.64 | 2.64 | 2.66 | 2.73 | 2.68 |
| C10' | 4.42 | 4.28 | 4.22 | 4.40 | 4.38 |
| C12' | 5.42 | 5.28 | 5.31 | 5.30 | ∼5.40 |
| C12'a | - | - | - | - | - |
| C13' | 1.45 | 1.42 | 1.42 | 1.42 | 1.44 |
| C14' | 1.05 | 1.18 | 1.08 | 1.19 | 1.09 |
| C15' | 0.86 | 0.83 | 0.83 | 0.99 | 0.87 |

**Table 2: ¹³C-Chemical shifts (ppm) of examples 2 and 3, as measured at 176.1 MHz, at 298 K in CDCl₃.**

| **C-Atoms** | **Example 2** | **Example 3** |
|---|---|---|
| C2 | 50.8 | 51.9 |
| C3 | 46.8 | 47.0 |
| C3' | 102.5 | 104.3 |
| C4' | 46.2 | 36.3 |
| C5' | 69.5 | 24.8 |
| C5'a | 58.6 | 43.9 |
| C6' | 37.0 | 41.1 |
| C7' | 34.9 | 69.8 |
| C8' | 21.6 | 28.8 |
| C8'a | 45.4 | 38.1 |
| C9' | 29.0 | 28.5 |
| C10' | 91.6 | 92.1 |
| C12' | 91.0 | 90.5 |
| C12'a | 79.5 | 79.8 |
| C13' | 25.5 | 25.8 |
| C14' | 21.3 | 16.3 |
| C15' | 13.4 | 13.2 |

### Preparation of Example 1 to 5

4-[(3R,6R,9R,10R,12R,12aR)-3,6,9-trimethyldecahydro-3,12-epoxy[1,2]dioxepino[4,3-i]iso-chromen-10-yl]thiomorpholine-1,1-dioxide (Example A)

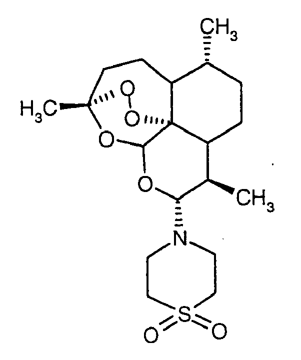

Example A is incubated with Human liver microsomes. Example 1 to 5 can be isolated after incubation.

Similar results can be achieved be using liver microsomes from Rhesus monkey, Beagle dog, Himalaya rabbit, Wistar rat (male), NMRI mouse, and CD-1 mouse.

### Incubation of Example A with human liver microsomes

Human liver microsomes 452161 lot: 22 are purchased from Gentest, Woburn, USA.

The incubation buffer consists out of 50 mM K₂HPO₄ + 1 mM EDTA (adjusted on pH 7.4) at 37°C, 1 mM NADP, 5 mM Glucose-6-Phosphate, and 1.5 U/ml Glucose-6-Phosphate Debydrogenase. The substrate, Example A dissolved in acetonitrile/water (1:1), is added to the solution to give a final concentration of 20 µM in a total incubation volume of 10 ml with a protein concentration of 0.5 mg/ml. The incubation is shaken at 37°C and 1200 RPM for 30 min and terminated by addition of 10 ml acetonitrile to 10 ml incubation. The samples are centrifuged and the supernatant is concentrated for preparative HPLC. For a production in a larger scale, up to 10 incubations with 10 ml volume each are performed in parallel.

### B. Evaluation of physiological activity

The *in vitro* effect of the compounds according to the invention can be demonstrated in the assays as described in WO 00/04024.

### C. Operative examples relating to pharmaceutical compositions

The compounds according to the invention can be converted into pharmaceutical preparations as follows:

### Tablet:

### Composition:

100 mg of the compound of Example 1, 50 mg of lactose (monohydrate), 50 mg of maize starch (native), 10 mg of polyvinylpyrrolidone (PVP 25) (from BASF, Ludwigshafen, Germany) and 2 mg of magnesium stearate.

Tablet weight 212 mg, diameter 8 mm, curvature radius 12 mm.

### Preparation:

The mixture of active component, lactose and starch is granulated with a 5% solution (m/m) of the PVP in water. After drying, the granules are mixed with magnesium stearate for 5 min. This mixture is moulded using a customary tablet press (tablet format, see above). The moulding force applied is typically 15 kN.

### Orally administrable suspension:

### Composition:

1000 mg of the compound of Example 1, 1000 mg of ethanol (96%), 400 mg of Rhodigel (xanthan gum from FMC, Pennsylvania, USA) and 99 g of water.

A single dose of 100 mg of the compound according to the invention is provided by 10 ml of oral suspension.

### Preparation:

The Rhodigel is suspended in ethanol and the active component is added to the suspension. The water is added with stirring. Stirring is continued for about 6h until the swelling of the Rhodigel is complete.

## Claims

1. A compound of the formula 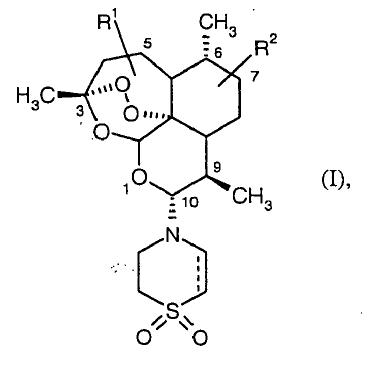 in which
..... represents a single or a double bond,
R¹ represents hydrogen or hydroxy,
and
R² represents hydrogen or hydroxy,
wherein
..... represents a double bond if R¹ and R² are hydrogen.
or one of its salts, its solvates or the solvates of its salts.

2. The compound according to Claim 1, wherein
..... represents a single or a double bond,
R¹ represents hydroxy,
and
R² represents hydrogen,
or
..... represents a single or a double bond,
R¹ represents hydrogen,
and
R² represents hydroxy,
or
..... represents a double bond,
R¹ represents hydrogen,
and
R² represents hydrogen.

3. The compound according to Claim 1 or 2, wherein R¹ represents hydroxy and the hydroxy-group is linked to the 5 position.

4. The compound according to Claim 1 or 2, wherein R² represents hydroxy and the hydroxy-group is linked to the 7 position.

5. A compound of the formula
4-[(3R,6R,9R,10R,12R,12aR)-3,6,9-trimethyldecahydro-3,12-epoxy[1,2]dioxepino[4,3-i]-isochromen- 10-yl]-3,4-dihydro-2H-1,4-thiazine-1,1-dioxide 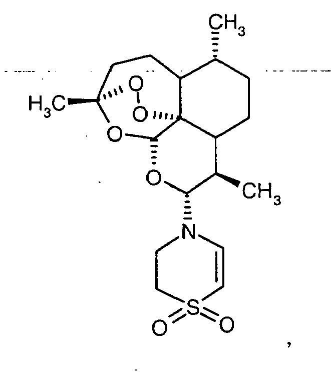 (3R,5S,6R,9R,10R,12R,12aS)-10-(1,1-dioxidothiomorpholin-4-yl)-3,6,9-trimethyldecahydro-3,12-epoxy[1,2]dioxepino[4,3-i]isochromen-5-ol 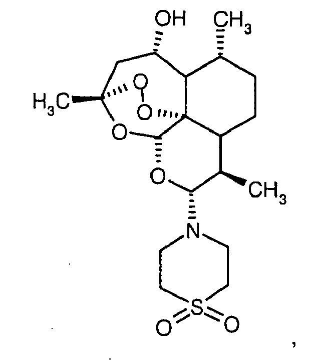 (3R,6S,7R,9R, 10R,12R,12aR)-10-(1,1-dioxidothiomorpholin-4-yl)-3,6,9-trimethyldecahydro-3,12-epoxy[1,2]dioxepino[4,3-i]isochromen-7-ol 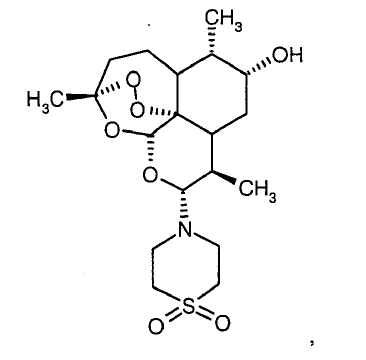 (3R,5S,6R,9R,10R,12R,12aS)-10-(1,1-dioxido-2,3-dihydro-4H-1,4-thiazin-4-yl)-3,6,9-trimethyldecahydro-3,12-epoxy[1,2]dioxepino[4,3-i]isochromen-5-ol 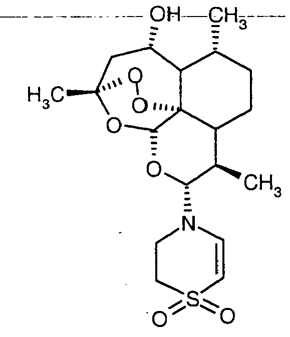 or
(3R,6S,7R,9R, 10R,12R,12aR)-10-(1,1-dioxido-2,3-dihydro-4H-1,4-thiazin-4-yl)-3,6,9-trimethyldecahydro-3,12-epoxy[l,2]dioxepmo[4,3-i]isochromen-7-ol

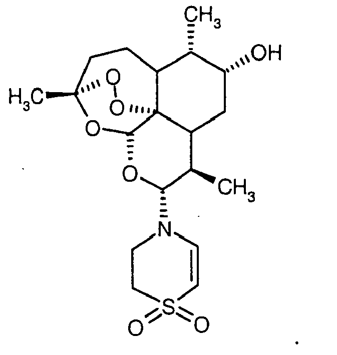

6. Process for preparing a compound according to Claim 1, **characterized in that** a compound of the formula 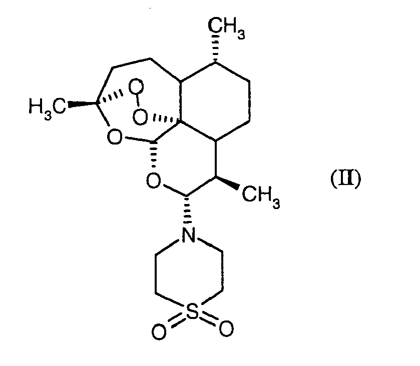 is incubated with liver microsomes of different species including man.

7. A compound according to any of Claims 1 to 5 for the treatment and/or prophylaxis of disorders.

8. A medicament containing at least one compound according to any of Claims 1 to 5 in combination with at least one pharmaceutically acceptable, pharmaceutically safe excipient.

9. Use of a compound according to any of Claims 1 to 5 for manufacturing a medicament for the treatment and/or prophylaxis of malaria.

10. The medicament according to Claim 8 for the treatment and/or prophylaxis of malaria.

11. A process for controlling malaria in humans and animals by administration of an effective amount of at least one compound according to any of Claims 1 to 5.
